# EUROPÄISCHE PATENTSCHRIFT

(11) **EP 1 951 183 B1**
(45) Veröffentlichungstag und Bekanntmachung des Hinweises auf die Patenterteilung: **19.10.2011**
(21) Anmeldenummer: 06818403.5
(22) Anmeldetag: 07.11.2006
(51) Int. Cl.: A61K 6/027, A61K 6/083

(54) **NANO-KRISTALLINE ERDALKALI - FÜLLSTOFFE ENTHALTENDE RESTAURATIONSMATERIALIEN**
RESTORING MATERIALS CONTAINING NANOCRYSTALLINE EARTH ALKALINE FILLERS
MATERIAUX DE RESTAURATION CONTENANT DES SUBSTANCES DE CHARGE A ALCALINO-TERREUX NANO-CRISTALLINS

(30) Priorität: 10.11.2005 DE 102005053705
(43) Veröffentlichungstag der Anmeldung: 06.08.2008
(73) Patentinhaber: S & C Polymer Silicon- und Composite-Spezialitäten GmbH, 25335 Elmshorn (DE)
(72) Erfinder: ENGELBRECHT, Jürgen, 22607 Hamburg (DE); PANTHER, Thomas, 3770 Kragerö (NO)
(74) Vertreter: Forstmeyer, Dietmar
(86) Internationale Anmeldenummer: PCT/EP2006/010658
(87) Internationale Veröffentlichungsnummer: WO 2007/054266

(56) Entgegenhaltungen:
- EP-A2- 0 002 831
- WO-A-01/01930
- WO-A-01/95863

## Beschreibung

Die Erfindung beschreibt nano-kristalline Erdalkali-Füllstoffe enthaltende Restaurationsmaterialien zur Verwendung an der Zahnsubstanz, die sich durch hohe Esthetik, hohe Härte, hervoragende Transparenz, gute Oberflächenpolierbarkeit, hohe Festigkeit und durch die Fähigkeit zur Freisetzung von Ionen in eine biologische Umgebung auszeichnen.

### Stand der Technik

Es besteht generell der Wunsch nach härtbaren restaurativen Dentalmaterialien, die günstige bioaktive Eigenschaften zeigen und zum Beispiel Fluorid-, Calzium- oder Hydroxyl-Ionen freisetzen können. Vorteilhaft an diesen Ionen ist, daß sie positive Auswirkungen in Bezug auf eine stärkere Remineralisierung haben und von ihnen eine kariostatische Wirkung ausgeht.

Es gibt viele restaurative Dentalmaterialien, die bekannterweise ionenabgebende Eigenschaften zeigen wie z. B. Glasionomerzemente, deren harzmodifizierten Varianten und Compomer-Füllungsmaterialien. (z.B. A. D. Wilson, J.W. McLean, Glass Ionomer Cement, Quintessence Publishers, Chicago,1988).

Restaurative Materialien dieser Arten zeigen einen hohen Grad an Ionen-Freisetzung wie von Calzium und Fluorid.
Negativ sind jedoch eine nicht ausreichende Säurefestigkeit und dauerhaft nicht ausreichende mechanische Festigkeit.

Andere Ansätze zur Lösung dieser Probleme sind der Einsatz von speziellen stark Ionen-abgebender Gläser, die in feinster Pulverform im µm-Größenbereich als Compositfüllstoffe eingesetzt sind, bei Kontakt mit Wasser eine hohe Fluoridabgabe aufweisen und bei guter Transparenz ästhetische Restaurationen ermöglichen (DE 197 57 645).

Dort verwandte Gläser mit "Invertglasstruktur" enthalten in einem weit höheren Maße als normale Gläser Fluoride, die offenbar in dieser Glasstruktur z. B. als CaF₂ vorliegen.

Die Erz0.002 831 beschreibt opake Zahnfüllungsmaterialien, die als Weißpigment feinverteiltes Calciumfluorid enthalten. Calciumfluorid wird gelobt als zwar opak-machendes aber dennoch nicht die Lichthärtung verhinderndes Pigment. Die Teilchengröße des Calciumfluorids liegt im µm-Bereich. Über eine merkliche Fluorid-Freisetzung wird nichts berichtet.

In der EP 1 139 995 wird die Herstellung und die Verwendung von schwerlöslichen Calciumsalzen, unter anderen Calciumfluorid, in einer Teilchengröße im Nanometerbereich in Zahnpflegemitteln beschrieben. Calciumfluorid wird dort als nützlich zur Festigung des Zahnschmelzes und zur Kariesprophylaxe beschrieben, besonders, wenn es in feinster Verteilung eingesetzt wird.

Aufgabe der Erfindung war es, härtbare restaurative Dentalmaterialien zu finden, die günstige bioaktive Eigenschaften zeigen und zum Beispiel Fluorid-, Calzium- oder andere Erdalkaliionen freisetzen können - ohne daß diese stark opak machen -, die esthetische optische Eigenschaften aufweisen können und die mechanisch und chemisch dauerhaft im Munde belastbar sind.

Erfindungsgemäß gelöst wurde die Aufgabe durch dentale Restaurationsmaterialien basierend auf einem vernetzbaren Harz-System und / oder einem härtenden Säure-/Base-Zement-System, welches nanostrukturiertes Erdalkalifluorid, bevorzugt Strontiumfluorid, besonders bevorzugt Calciumfluorid enthält, wobei die Partikelgröße des Erdalkalifluorids mehrheitlich im Bereich von 2 bis 200 nm liegt.

### Beschreibung der Erfindung

Erfinderisch aufgabengemäß konnte gezeigt werden, daß bei einem Einsatz von nano-kristallinen Erdalkali-Füllstoffen (optional mit Zusätzen anderer Füllstoffe wie z. B. Gläsern oder microfeiner Kieselsäure) in härtbaren Restaurationsmaterialien entweder auf Basis von in Dentalwerkstoffen gebräuchlichen vernetzbaren Harz-Systemen oder Zement-Systemen oder auch Kombinationen hiervon eine gewünschte zahnähnliche Transparenz und eine hohe mechanische Belastbarkeit erreicht wird. Außerdem zeigen diese Materialien die erwünschte Fähigkeit zur Ionenabgabe und Ionenaufnahme.

Als härtbare restaurative Dentalmaterialien sollen im Rahmen der Erfindung solche Materialien bezeichnet werden, die für Zahnfüllungen, Inlays oder Onlays, Befestigungszemente, Verblendmaterialien für Kronen und Brücken, Materialien für künstliche Zähne, Dentin- und Schmelzbondings, Unterfüllungsmaterialien, Stumpfaufbaumaterialien, Wurzelfüllmaterialien oder sonstigen härtbare Materialien für die prothetische, konservierende und präventive Zahnheilkunde Verwendung finden.

Die Dentalmaterialien der vorliegenden Erfindung vermögen Fluorid- und Erdalkaliionen in ihre biologische Umgebung abzugeben und erfüllen die für Dentalmaterialien geforderten esthetischen und physikalischen Eigenschaften.

Die härtbaren Dentalmaterialien bestehen aus einer härtbaren Matrix (zum Einen als vernetzbare Harze und/oder zum Anderen als Säure-Base-Zemente) und Erdalkalifluoriden bzw. gegebenenfalls anderen Füllstoffen und gegebenenfalls anderen Zusätzen.

Zu einer wichtigen Gruppe mit Erfolg einsetzbarer Matrices aus der Gruppe vernetzbarer Harze gehören polymerisierbare, ethylenisch ungesättigten Monomere, vorzugsweise solche mit acrylischen und/oder methacrylischen Gruppen.

Insbesondere handelt es sich hierbei u. a. um Ester der Cyanoacrylsäure, (Meth)acrylsäure, Urethan(meth)acrylsäure, Crotonsäure, Zimtsäure, Sorbinsäure, Maleinsäure und Itaconsäure mit ein- oder zweiwertigen Alkoholen; (Meth)acrylamide wie z.B. N-isobutylacrylamid; Vinylester von Carbonsäuren wie z.B. Vinylacetat; Vinylether wie z.B. Butylvinylether; Mono-N-vinylVerbindungen wie N-Vinylpyrrolidon; und Styrol sowie seine Derivate. Besonders bevorzugt sind die nachfolgend aufgeführten mono- und polyfunktionellen (Meth)Acrylsäureester und Urethan(meth)acrylsäureester:
(a) Monofunktionelle (Meth)acrylate
   Methyl(meth)acrylat, n- oder i-Propyl(meth)acrylat, n-, i- oder tert.-Butyl(meth) acrylat und 2-Hydroxyethyl(methacrylat.
(b) Difunktionelle (Meth)acrylate
   Verbindungen der allgemeinen Formel: 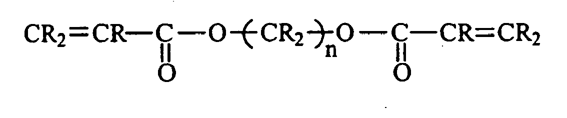 worin R Wasserstoff oder Methyl ist und n eine positive ganze Zahl zwischen 3 und 20, wie
   z. B. Di(meth)acrylat des Propandiols, Butandiols, Hexandiols, Octandiols, Nonandiols, Decandiols und Eicosandiols,
   Verbindungen der allgemeinen Formel: 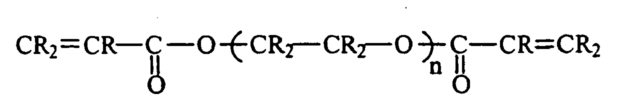 worin R Wasserstoff oder Methyl ist und n eine positive ganze Zahl zwischen 1 und 14, wie
   z. B. Di(meth)acrylat des Ethylenglycols, Diethylenglycols, Triethylenglycols, Tetraethylenglycols, Dodecaethylenglycols, Tetradecaethylenglycols, Propylenglycols, Dipropylglycols und Tetradecapropylenglycols; und Glycerindi(meth)acrylat, 2,2'-Bis(4(3'-methacryloyl-oxy-2'-hydroxypropoxy)phenyl]propan) oder Bis-GMA, Biphenol-A-dimethacrylat, Neopentylglycoldy(meth)acrylat, 2,2'-Di(4-meth acryloxypolyethoxyphenyl)-propan mit 2 bis 10 Ethoxygruppen pro Molekül und 1,2-Bis(3-methacryloxy-2 hydroxy-propoxy)butan.
(c) Tri- oder mehrfachfunktionelle (Meth)acrylate
   Trimethylolpropantri(meth)acrylate und Pentaerythritoltetra(meth)acrylat.
(d) Urethan(meth)acrylate, welche üblicherweise durch Umsetzung von 2 Mol hydroxylgruppenhaltigen (Meth)acrylatmonomer mit einem Mol Diisocyanat erhalten werden.
e) Siloxan-basierte Mono-, Di- oder Multiacrylate Auch Siloxan-basierte mono-, di- oder Multiacrylate sind in diesem Zusammenhang von Bedeutung.

Die genannten Monomeren werden entweder allein oder in Form einer Mischung von mehreren Monomeren verwendet.

Zu ganz besonders vorteilhaft im erfindungsgemäßen Restaurationsmaterial eingesetzten Monomeren gehören vor allem 2,2-Bis-4(3-methacryloxy-2-hydroxypropoxy)-phenylpropan (Bis-GMA), 3,6-Dioxaoctamethylendimethacrylat (TEDMA) und/oder 7,7,9-Trimethyl-4, 13-dioxo-3, 14-dioxa-5, 12-diazahexadecan-1, 16-dioxy-dimethacrylat (UDMA).

Die Monomeren können auch basische Gruppen, wie z.B. Amine, oder auch saure Gruppen, wie sie z.B. in US 4,806,381 beschrieben sind, tragen.

Das Material kann je nach Art des verwendeten Katalysators heiß, kalt und/oder durch Licht polymerisierbar sein. Als Katalysatoren für die Heißpolymerisation können die bekannten Peroxide wie Dibenzoylperoxid, Dilauroylperoxid, tert.-Butylperoctoat oder tert.-Butyl-perbenzoat eingesetzt werden, aber auch Azo-bis(isobutyroethylester), Benzpinakol und 2,2'-Dimethylbenzpinakol sind geeignet.

Als Katalysatoren für die Photopolymerisation können z.B. Benzophenon und seine Derivate sowie Benzoin und seine Derivate verwendet werden. Weitere bevorzugte Photosensibilisatoren sind a-Diketone wie 9,10-Phenanthrenchinon, Diacetyl, Furil, Anisil, 4,4'-Dichlorbenzil und 4,4'-Dialkoxybenzil. Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Katalysatoren für die Kaltpolymerisation werden Radikale liefernde Systeme, z.B. Benzoyl- bzw. Lauroylperoxid zusammen mit Aminen wie N,N-Dimethyl-sym.-xylidin oder N,N-Dimenthyl-p-toluidin verwendet. Es können auch dual härtende Systeme zur Katalyse verwendet werden, z.B. Photoinitiatoren mit Aminen und Peroxiden. Als Photokatalysatoren kommen auch Mischungen aus UVlichthärtenden und im Bereich des sichtbaren Lichts härtenden Katalysatoren in Betracht.

Die Menge dieser Katalysatoren im Dentalwerkstoff liegt üblicherweise zwischen 0,01 bis 5 Gew.-%.

Zu einer anderen Gruppe von mit Erfolg einsetzbarer Harzmatrices gehören ringöffnende Monomere, die unter Ringöffnung polymerisieren bzw. polyaddieren.

Beispiele umfassen organische Verbindungen, welche z.B. einen Oxiranring, d.h. 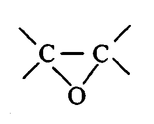 aufweisen, welcher durch Ringöffnung polymerisierbar ist. Solche Materialien werden allgemein als Epoxide bezeichnet; sie umfassen monomere Epoxidverbindungen sowie Epoxide vom polymeren Typ und können aliphatische, cycloaliphatische, aromatische oder heterocyclische Verbindungen darstellen. Diese Materialien weisen im allgemeinen durchschnittlich mindestens eine polymerisierbare Epoxidgruppe pro Molekül auf, vorzugsweise mindestens etwa 1,5 polymerisierbare Epoxidgruppen pro Molekül. Die polymeren Epoxide umfassen lineare Polymere mit endständigen Epoxidgruppen (z.B. einen Diglycidylether eines Polyoxyalkylenglycols), Polymere mit Oxiraneinheiten im Molekülgerüst (z.B. Polybutadien-polyepoxid) sowie Polymere mit am Gerüst anhängenden Epoxidgruppen (z.B. ein Glycidylmethacrylat-Polymer oder -Copolymer). Diese Epoxide können reine Verbindungen oder Mischungen sein, welche eine, zwei oder mehrere Epoxidgruppen pro Molekül enthalten.

Geeignete epoxidhaltige Materialien umfassen Materialien, welche Cyclohexenoxid-Gruppen enthalten, wie z.B. die Epoxycyclohexancarboxylate; typische Beispiele sind 3 , 4-Epoxycyclohexyl-methyl-3 , 4-epoxycyclohexancarboxylat, 3 , 4-Epoxy-2-methylcyclo-hexylmethyl-3 ,4-epoxy-2-methylcyclohexancarboxylat sowie Bis-(3 , 4-epoxy-6-methylcyclohexylmethyl) adipat . Für eine detailliertere Auflistung verwendbarer Epoxide dieses Typs wird auf US-Pat. Nr. 3,117,099 verwiesen.

Weitere verwendbare Epoxidharze sind Silicone mit Epoxidfunktionalität, insbesondere Cyclohexylepoxid-Gruppen, insbesondere solche mit einem Silicon-Grundgerüst. Beispiele sind UV 9300, UV 9315, UV 9400 und UV 9425, welche alle von GE Bayer Silicones geliefert werden.

Ganz besonders geeignet und besonders biokompatibel sind Monomere mit Epoxifunktionen und organischen Grundgerüsten, linear oder cyclisch, wie sie in DE 19860361 und PCT/FR99/02345 als Monomere als auch mit ihren entsprechenden Startersystemen für licht-, heiß- oder kalthärtende Mischungen beschrieben sind.

Hydroxylhaltige Materialien können zugesetzt werden, die Zugabe von Vinylether-Verbindungen zu dem Epoxidharz kann nützlich sein.

Weitere ringöffnende Monomere können Verbinbungen der Orthokohlensäure sein, als Beispiel sei hier die Grundstruktur der Spiro-Orthokohlensäure erwähnt (Beyerlei et. al. US 5,556,896):

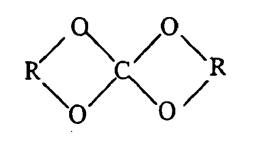

Auch Ormocer-Matrices, wie sie in WO 92/16571, DE 4133494 oder DE 10016324 beschrieben sind, sind geeignet.

Zu einer anderen neuerlich einsetzbaren Gruppe härtbarer Monomeren für restaurative Zwecke gehören auch Monomere auf Siliconbasis.

Z.B.,kann eine Harzmatrix, wie sie in der DE 3915592 A1 für einen Dentalzement beschrieben ist, genommen werden, die aus einer Härtungsflüssigkeit, und zwar einem mit Carboxylgruppen modifizierten Silikonöl, und einem Härtungsbeschleuniger, und zwar einem Metalloxid und/oder einem Metallhydroxid besteht.

Z.B. kann eine Harzmatrix, wie sie in der US 3 127363 A beschrieben ist, geeignet sein, in der ein Organosiloxan (A) der allgemeinen Formel XO-Si(R)₂-(O-SiR₂] ₙ-O-Si (R) ₂-OX mit einem trifunktionellen Vernetzungsmittel (B), beispielsweise der Formel RSi(OH)_{3,} vernetzend kondensiert werden.

Es kann auch eine zweikomponentige Harzmatrix geeignet sein, bei der eine der beiden Komponenten (Komponente I) aus einem oder mehreren Silikonölen besteht oder diese umfaßt, die mindestens zwei SiH-Gruppen aufweisen, und die andere der beiden Komponenten (Komponente II) aus einem oder mehreren Silikonölen besteht oder diese umfaßt, die mindestens zwei Vinylgruppen aufweisen, und eine der beiden Komponenten zusätzlich einen Katalysator für eine Additionsreaktion oder eine Additionsvernetzung der beiden Komponenten I und II enthält.

Mit solchen Mischungen lassen sich z.B. Wurzelkanalfüllungs Mischungen herstellen, wie sie in EP 0864 312 B1 beschrieben sind und welche z.B. durch Zugabe von Nano-Erdalkalifluoriden in diese einen Fluortransport in feinste Dentinkanälchen bewirken können.

Eine andere bedeutende Gruppe in diesem Zusammenhang betrifft Materialien, deren härtbare Matrix auf Zementsystemen beruht (Härtungsreaktion von Säuregruppen-Komponenten mit basischen Komponenten). Klassische Zemente im Dental- und Medizinbereich bestehen hauptsächlich aus Säuren und reaktiven alkalischen Gläsern oder Metalloxiden. Der Begriff "klassische" Zemente bedeutet harzfreie Zemente. Harzmodifizierte Versionen von diesen Zementen wurden ohne weiteres durch das Vermischen mit polymerisierbaren,Harzen zubereitet. Zemente enthalten mehrere charakteristische Gruppen:

Die klassischen dentalen Polyalkenoat-Zemente enthalten fein zermahlenes Fluoroaluminiumsilikat-Glaspulver (oder basische Oxide), Polyalkenoatsäuren und Wasser. Die Bildung von wasserunlöslichen dentalen Polyalkenoatzementen aus den wasserbasierten Formulierungen findet in mehreren Stufen und über längere Zeit statt. Zusammenfassend greift die Polyalkenoatsäure das Fluoroaluminiumsilikat-Pulver in Gegenwart von Wasser an den säurezugänglichen Stellen an und resultierend werden Ionen (speziell Erdalkalimetallionen und Aluminiumionen) freigesetzt. Diese freigesetzten Metallionen gehen mit Alkenoat-Gruppen der Polyalkenoatsäuren eine Ionenbindung ein und bilden eine vernetzte Struktur. Auf diese Weise wird der salzartige Zement zu einem wasserunlöslichen harten Material. Die Aushärtung bzw. Abbindung des Polyalkenoatzementes zu diesem wasserunlöslichen Material findet in den ersten Minuten sehr schnell statt. Mit zunehmender Abbindung verlangsamt sich die Reaktion über mehrere Stunden bis mehrere Tage. Während dieser Zeit ist der Zement noch nicht speichelresistent.

Passende vernetzbare Säuren von Polyalkenoatsäuren sind zum Beispiel Polyacrylsäure, Polyitaconsäure, Polymaleinsäure, Milchsäure, Polyvinylsulfonsäure, Polystyrensulfonsäure, Polyschwefligesäure, Polyvinylphosphonsäure und Polyvinylphosphorsäure oder deren Copolymere. Die Hauptbestandteile der Polyalkenoat-Zemente können zum Beispiel Fluoroaluminiumsilikat-Glaspulver enthalten. Diese bestehen aus 10 bis 25 Gew.-% A1, 5 bis 30 Gew.-% Si, 1 bis 30 Gew.-% F, 0. bis 20 Gew.-% Sr, 0 bis 20 Gew.-% Ca, und 0 bis 10 Gew.-% Alkalimetallionen (Na⁺, K⁺, usw.), bezogen auf das Gesamtgewicht des Glases. Die Zubereitung erfolgt durch das Vermischen der Einzelkomponenten und Verschmelzen der Mischung. Abschließend wird das Material abgekühlt und bis zu einer mittleren Partikelgröße von 0.2 bis 20 µm zermahlen.

Bei Einsatz basischer Oxide zur Herstellung der wasserbasierten Polyalkenoat-Zemente werden Zinkoxid bzw. Zinkoxid und deaktiviertes Zinkoxid/Magnesiumoxid bevorzugt verwendet. Ebenso bevorzugt sind die neueren zink-basierten Dentalmischungen aus EP No. EPO883586. Andere basische Oxid-Zemente enthalten solche Oxide von Be, Cu, Mg, Ca, Sr und Ba und Kombinationen hiervon.

Die "klassischen" dentalen Silikat-Zemente enthalten fein zermahlenes Fluoroaluminiumsilikat-Glaspulver (oder basische Oxide), Phosphorsäure und Wasser.

Die "klassischen" dentalen Phosphat-Zemente enthalten fein zermahlenes modifiziertes Zinkoxidpulver (oder andere basische Oxide), Phosphorsäure und Wasser.

Andere klassische Dentalzemente können auch Zemente sein, die sich zum großen Teil aus Zinkoxid oder anderen Metalloxiden und aus schwachen organischen Säuren oder phenol-haltigen Verbindungen (z. B. Eugenol) zusammensetzen.

Die Konsistenz der Dentalzemente kann pulverförmig, flüssig oder pastös sein. Zusätzlich zu der Zusammensetzung der Dentalzemente entsprechend der vorliegenden Erfindung können UV-lichtabsorbierende Stoffe, Weichmacher, Antioxidantien, Bakterizide, Tenside, usw. zugefügt werden.

Wichtige Bestandteile der o. g. härtenden Materialien oder Zementsysteme sind nanostrukturierte Erdalkalifluoride MF₂
wobei M die Erdalkalikation Ca²⁺, Mg²⁺ Sr²⁺ oder Ba²⁺ und F das Fluoridanion darstellen. Die Partikelgröße des Erdalkalifluorids liegt mehrheitlich im Bereich von 2 bis 200 nm.

Besonders wichtig ist das Calciumfluorid. Calcium und Fluorid sind wesentliche Bestandteile des Apatits der Zahnhartsubstanz (Ca₁₀(PO₄)₆(OH,F)₂.

Die Gegenwart von Strontium im Apatit des Zahns wird für den zahnheilkundlichen Nutzen in Verbindung mit einem möglichen karieshemmenden Effekt als wichtig erachtet (zusätzlich zu dem vermuteten Effekt der Reduzierung der Dentinempfindlichkeit), und es verleiht dem Apatit eine geringere Löslichkeit und eine höhere Resistenz gegenüber thermischen Einflüssen. Zusätzlich wird die Röntgenopazität erhöht.

Nanokristalline Materialien sind im allgemeinen künstlich hergestellte Materialien, welche durch zusammenhängende Phasen oder durch granulare Strukturen und einer Länge von gewöhnlich weniger als 200 nm charakteriseirt sind. In Abhängigkeit von der Anzahl der Dimensionen, in denen diese Materialien eine Nanostruktur aufweisen, unterscheidet man zwischen (i) nulldimensionalen, (ii)eindimensionalen, (iii) zweidimensionalen und (iv) dreidimensionalen Materialien (R. W. Siegel, in Materials Science in Technology, Vol. 15: Processing of Metals and Alloys, R. W. Chan, 583 (1991)).

Die spezifischen Eigenschaften der nanokristallinen Materialien resultieren aus drei grundlegenden Merkmalen, nämlich (i) dem atomaren Größenbereich von 200 nm, (ii) dem hohen Anteil von an den Grenzflächen beteiligten Atomen und (iii) den Wechselwirkungen zwischen den einzelen Teilbereichen.

Bei Partikelgrößen im Nanobereich findet sich ein hoher Anteil an Oberflächenmolekülen zur Gesamtzahl der Moleküle eines Teilchens. In einem Material mit einer durchschnittlichen Partikelgröße von 10-15 nm sind 15-50 % der Atome an den "Korngrenzflächen" beteiligt.

Weil die Anzahl der Grenzflächen in nanokristallinen Materialien sehr viel höher ist, als in konventionellen Materialien, läßt sich durch eine geeignete Kontrolle im Laufe der Synthese der Materialien durch die Natur der Grenzflächen die Natur der Wechselwirkungen zwischen den Grenzflächen aller beteiligten Phasen bewirken.

Die Wechselwirkung zwischen Nano-Erdalkalifluorid in einer polymeren Matrix und seiner biologischen Umgebung ist sehr viel intensiver als die Wechselwirkung, die man erhält wenn es sich um Erdalkalifluoride im µm-Bereich in dieser Matrix handelt. Wie diese Erfindung zeigt, haben sich folglich Materialien auf der Grundlage von Nano-Erdalkalifluoriden als sehr brauchbare dentale Restaurationsmaterialen erwiesen.

Eine weitere wichtige Eigenschaft der Nano-Dimensionen der Nano-Erdalkalifluoride ist, daß eine Lichtbrechung im sichtbaren Bereich, d. h. für Licht der Wellenlänge 400 - 900 nm, nicht mehr stattfindet. Die Nano-Partikel sind zu klein, um mit dem deutlich langwelligerem Licht wechselzuwirken.

Dadurch führen auch Füllstoffe wie Erdalkalifluoride mit Brechungsindices von 1,37 - 1,47 in üblichen polymerisierten Harzmatrices mit Brechnungindizes von 1,49 bis 1,54 , die sonst zu weiß-opaken Mischungen und weiß-opaken ausgehärteten Feststoffen führen, im Falle von Nanopartikelgrößen zu recht transparenten, für dentale esthetische Anwendungen geeignete Mischungen.

Die Nano-Partikelgröße der im Dentalwerkstoff gemäß der Erfindung einzusetzenden Erdalkalifluoride liegt mehrheitlich im Bereich von 2 - 200 nm, bevorzugt zwischen 2 und 90 nm.

In einer bevorzugten Ausführung der Erfindung sind die Nano-Erdalkalifluoridpartikel oberflächenbehandelt, um eine bessere Einarbeitbarkeit in die Harzmatrix sowie bessere mechanische Eigenschaften der polymerisierten Produkte zu erreichen.

Zum Beispiel können Erdalkalifluorid-Füllstoffe mit mono- oder mehrfachfunktionellen Methacrylatestern der Phosphor-, Phosphon- oder Carbonsäuren oder Kombinationen daraus oberflächenbehandelt sein. Besonders bevorzugt sind die Ester des Hydroxylethylmethacrylats und des Glycerindimethacrylats. Auch polymethacrylierte Polyvinylphosphorsäuren oder polymethacrylierte Polyvinylphosphonsäuren oder polymethacrylierte Polycarbonsäuren können zur Oberflächenmodifikation geeignet sein. Weitere vorteilhafte Reaktionspartner für eine Oberflächenbehandlung können entsprechende Phosphorsäureester, Phosphonsäureester oder Carbonsäureester mit Vinylgruppen sein. Maleinsäure kann ebenso hilfreich sein. Die Wahl des oberflächlichen Vorreaktionsmittels hängt von den reaktiven Gruppen der gewählten Harzmatrix ab, z. B. Methacrylate für Methacrylatharze, Vinylethergruppen oder Epoxide für Epoxidharze, Vinyl- oder SiH-Gruppen für additionsvernetzende Vinylsiloxanmatrices.

Damit soll eine für den jeweiligen Anwendungszweck vorteilhafte organische Modifizierung der Partikel an der Oberfläche möglich gemacht werden, die dann durch kovalente Bindungen für eine höhere mechanische Festigkeit sorgen soll.

Die Wahl des Oberflächenbehandlungsmittels kann aber auch so gewählt werden, daß nur die Dispersivität des Nano-Erdalkalifluorids sowie der Füllgrad mit diesem erhöht wird, ohne daß dies in einer mechanischen Verstärkung resultiert. Beispiele dieser Art sind eine Behandlung mit Phosphatsalzen oder mit Nicht-Organosilanen.

Eine weitere Methode ist das Aufbringen einer Schicht SiO₂ oder ZrO₂ ebenfalls im Nanometermaßstab und anschließendes Behandeln mit einem reaktiv-funktionellem Silan wie z. B. Methacryl-, Glycidyl-, Amino- oder Vinylorganosilanen soweit dadurch die Eigenschaften des Ionenaustausches nicht zu sehr beeinflußt werden.

Der Nano-Erdalkalifluorid-Füller kann auch in eine der oben beschriebenen Harzmatrices eingearbeitet, polymerisiert und genahlen werden und dann als Füllstoff eingesetzt werden und zwar sowohl mit, als auch ohne die oben beschriebene Art der Oberflächenbehandlung.

Die Nano-Erdalkalifluorid-Füller sind erfindungsgemäß im Dentalwerkstoff in einer Menge enthalten, die einerseits ausreicht, einen Ionenaustausch mit der biologischen Umgebung zu erlauben und andererseits die Bewahrung guter mechanischer Eigenschaften sicherstellt. Vorteilhaft ist es daher, wenn der Gewichtsanteil 1 bis 70 Gew.-%, insbesondere 1 bis 30 % bezogen auf den Restaurationswerkstoff, beträgt. Für eine hohe mechanische Festigkeit besonders zweckmäßig ist eine Abwandlung, worin der Gewichtsanteil 1 bis 15 Gew.-%, bezogen auf das Gesamtgewicht des Dentalmaterials, beträgt.

Neben der essentiellen und charakteristischen Füllstoffkomponente Nano-Erdalkalifluorid kann der Dentalwerkstoff weitere Füllstoffe als optionale, aber dennoch sehr vorteilige und nicht minder bevorzugte Komponenten aufweisen.

Weitere Füllstoffe können gemahlene Pulver aus SiO₂ und/oder Glaskeramik und/oder Glas, und/oder Mikrokugeln einer durchschnittlichen Teilchengröße von 0,2 bis 40 µ, anorganische und/oder organische Fasern, andere Nano-Füllstoffe wie SiO₂ und Metalloxide in Nanoteilchengröße, agglomerierte Nano-Füllstoffe, ungefüllte oder gefüllte Splitterpolymerisate, lonen-freisetzende Gläser oder Reaktionsprodukte davon oder andere Füllstoffe mit Wirkstoff-freisetzenden Eigenschaften oder zur Erhöhung der Röntgenopazität sein.

Als bevorzugte gemahlene Pulver werden Ba-, Sr- oder Li-Al-Silikatgläser mit einer mittleren Korngröße im Bereich von 0,2 bis 2,0 µm und Brechungsindices von 1,49 bis 1,53 eingesetzt.

Bevorzugte Mikrokugeln sind Füllstoffe wie sie beispielsweise in DE-A 32 47 800 beschrieben sind. Die durchschnittliche Primärteilchengröße liegt im Bereich von 0,1 bis 1,0 µm, insbesondere bei 0,15 bis 0,5 µm.

Die anorganischen oder organischen Fasern können Glas-, Aluminiumoxid-, Polyethylenoxid- oder Carbonfasern sein und können einer gerichteten Verstärkung dienen.

Andere Nanofüllstoffe bzw. agglomerierte Nanofüllstoffe auf SiO₂ Basis, ggf. mit Metalloxiden beschichtet, oder auf Metalloxidbasis sind polydispers, monodispers und können auch als Mischung von polydispersen und monodispersen Sphären vorliegen. Sie werden beispielsweise in W. Stöber et al. in J. Colloid and Interface Science 26,62(1968) und 30, 568 (1969), US-Patent 3,634,588, EP 0 216 278, EP 0 275 688 beschrieben.

Es können auch gesinterte Füllstoffe als Zusatz in Betracht kommen, wie sie z.B. in EP 0.113 926 beschrieben sind.

Gegebenenfalls können noch weitere Füllstoffe zur Erzielung einer erhöhten Röntgenopazität eingesetzt werden, wobei deren mittlere Primärteilchengröße 5,0 µm nicht übersteigen sollte. Solche Füllstoffe sind z.B. in der DE-OS 35 02 594 beschrieben.

Die Füllstoffe können ebenfalls zur besseren Einarbeitbarkeit oder für eine bessere Mechanik oberflächenbehandelt sein nach Methoden ähnlich wie bei den Nano-Erdalkalifluoriden erwähnt.

Alternativ hierzu ist es auch möglich, erfindungsgemäße und/oder andere Nanofüllstoffe bzw. agglomerierte Nanofüllstoffe oder andere feinstteilige Füllstoffe vor der eigentlichen Einarbeitung in den Dentalwerkstoff in einer Harzmatrix zu polymerisieren und anschließend feinzumahlen. Gegebenenfalls können zur Einstellung der Viskosität geringe Mengen an gegebenenfalls silanisierter mikrofeiner, pyrogener oder naßgefällter Kieselsäure in den Dentalwerkstoff eingearbeitet werden, höchstens jedoch 50 Gew.-%, bezogen auf den Dentalwerkstoff. Bevorzugt sind 1 - 25 %, besonders bevorzugt 3 -10 Gew.-%.

Als weitere Bestandteile können auch andere bioaktive oder antibiotische Substanzen zugegeben sein, wie z. B. in PCT/US 96/17871 beschrieben.

Die erfindungsgemäß beschriebenen Restaurationsmaterialien können aufgrund ihrer hervorragenden optischen Eigenschaften im sichtbaren Bereich der Mundhöhle und damit in direktem Kontakt zum Speichel eingesetzt werden.

Der Erdalkalifluoridanteil des restaurativen Materials kann damit an den Speichel und an den umgebenden Zahnschmelz Ionen abgeben (u. a. Fluorid, Calcium). Der u. a. erfindungsgemäß erstrebte Effekt, nämlich der Austausch von Ionen mit der biologischen Umgebung des Dentalwerkstoffs bei gleichzeitig hoher Transparenz und Härte des bioaktiven Werkstoffs läßt sich mit Nano-Erdalkalifluoriden als Füllstoff in polymerierbaren Monomeren und in Zementen erreichen.

So kann der erfindungsgemäße Füllstoff in typischen dentalen Formulierungen wie leicht gefüllten Harzen, Compositen, Ormoceren, Giomeren, Compomeren, Resin-Reinforced-Zementen, klassischen Zementen (z. B. klassische Polyalkenoat Zemente, Silikat-Zemente, Phosphat-Zemente), Oxiranen, Siloranen, oder Siliconen eingesetzt werden, welche als Zahnfüllungen, Inlays oder Onlays, Befestigungszemente, Verblendmaterialien für Kronen und Brücken, Materialien für künstliche Zähne, Dentin- und Schmelzbondings, Unterfüllungsmaterialien, Stumpfaufbaumaterialien. Wurzelfüllmaterialien oder sonstigen härtbare Materialien für die prothetische, konservierende und präventive Zahnheilkunde Verwendung finden.

Nachfolgend wird die Erfindung anhand von Ausführungsbeispielen und Vergleichsbeispielen weitgehend erläutert.

### Beispiel 1:

### Herstellung nanokristallinen Calciumfluorids.

Nanokristallines Calciumfluorid wurde aus einer ternären Microemulsion kristallisiert. Dazu wurde eine wässrige Phase enthaltend KF (Merck, Darmstadt, Deutschland) in eine Mischung von Brij 35® (Polyethylenglykol-laurylether, J.T. Baker, Deventer/Holland) und Octan (Sigma-Aldrich, Schnelldorf, Deutschland) im festen Gewichtsverhältnis 3 : 7 emulgiert. Die Microemulsion wurde bei 30°C mit 50 Gew.% 1,5 Mol KF kräftig gerührt, um eine Microemulsion zu erhalten. Wiederum unter heftigem Rühren wurde langsam eine stöchiometrische Menge einer wässrigen Lösung enthaltend 0,75 Mol CaCl₂ 6H₂O (Riedel de Häen) zugegeben und 24 h bei 30°C ruhen gelassen. Der Rückstand wurde abzentrifugiert, zweimal mit Alkohol und eimal mit Wasser im Ultraschallbad (*Bandelin Sonorex)* mit Hilfe eines *Ultra Turrax® T 25 basic* bei 24.000 U/min 5 min dispergiert, dann gewaschen und jeweils abzentrifugiert. Anschließend wurde das feuchte Calciumfluorid 4h Ultraschall-behandelt und sofort danach 96h gefriergetrocknet (Wassergehalt < 800ppm).

Das ultrafeine Pulver wurden auf Kristallinität, Korngröße und Identität untersucht.

Röntgendiffrakrometerdiagramme zeigen neben einem hohen Grad an Kristallinität identische Beugungsmuster zu einer entsprechenden Calciumfluorid-Referenz.

Die mit einem Partikelsizer (Horiba LA 920,Retsch Technologie,Deutschland) ermittelten Partikelgrößen lagen bei 110 - 150 nm.

Die aufgenommenen IR-Spektren des in Beispiel 1 synthetisierten Nano-Calciumfluorides stimmen mit einer Vergleichssubstanz (CaF₂ p.A., Merck, Darmstadt) überein.

### Beispiel 2:

Es wurde ein Composit mit einem Gehalt von 12% an Nano-Calciumfluorid hergestellt. Dazu wurde in eine Harzmatrix auf Basis von lichthärtbaren Dimethacrylaten die verschiedenen Mengen Füllstoffen zugesetzt. Aus den erhaltenen Pasten wurden durch Lichthärtung in einem Lichthärtegerät Dentacolor XS (Kulzer, Deuschland) Probekörper hergestellt und diese auf verschiedene Eigenschaften untersucht.

Die verwendete Harzmatrix wurde hergestellt aus

| | |
|---|---|
| 10 | Teilen Triethylengly,coldimethacrylat |
| 10 | Teilen Bis-GMA |
| 10 | Teilen Urethandimethacrylat |
| 0,05 | Teilen Campherchinon |
| 0,05 | Teilen Dimethylaminoethylmethacrylat |

In 20 Teile der Harzmatrix wurden 2 Teile Aerosil 202 und die folgenden Füllstoffe eingearbeitet:

### C MH (Vergleichsbeispiel)

| | |
|---|---|
| 78 Teile | Bariumaluminiumborsilikatglas 0,7 µm, methacrylsilanisiert |
| 0 Teile | Nano-Calciumfluorid nach Beispiel 1 |

### C MH/CaF(erfindungsgemäß)

| | |
|---|---|
| 66 | Teile Bariumaluminiumborsilikatglas 0,7 µm, methacrylsilanisiert |
| 12 | Teile Nano-Calciumfluorid nach Beispiel 1 |

Zur Bewertung der physikalischen und Fluoridfreisetzungs-Eigenschaften dieses Nano-Calciumfluorid-gefüllten Komposites wurden neben der Vergleichsprobe zusätzlich ein harzmodifizierter Zement (LC Vitrofil, Dental Fillings, Brasilien) und ein Compomer (Ionomolar, Dr. Ihde, Deutschland) herangezogen,und in Tabelle 1 wiedergegeben.

**Tabelle 1**

| **Prüfverfahren** | **Composit C MH** | **Composit C MH/CaF (erfindungsgemäß)** | **Harzmodifizierter Zement** | **Compomer** |
|---|---|---|---|---|
| Durchhärtetiefe [mm], 30 sec. | 6,1 | 6,0 | 7,3 | 6,8 |
| Opazität [%] | 81 | 82 | 83 | 86 |
| Biegebruchfestigkeit [MPa] | 162 | 168 | 73 | 134 |
| Elastizitätsmodul [GPa] | 10,6 | 14,4 | 9,85 | 14,4 |
| Barcolhärte | 80 | 79 | 70 | 81 |
| Wasseraufnahme [µg/mm³] | 22,5 | 72 | 92 | 28,3 |
| Wasseröslichkeit [µg/mm³ | 1,9 | 11,8 | 10,5 | 3,1 |

Tabelle 1 zeigt, dass
1. die Durchhärtetiefe gut und vergleichbar mit dem Compositmaterial C MH, harzmodifiziertem Zement und Compomermaterial ist,
2. die Opazität gut ist, um esthetische Restaurationsmaterialien herzustellen zu können und vergleichbar mit den genannten anderen Zahnfüllungsmaterialien,
3. die Biegebruchfestigkeit und Elastizitätsmodul denen vom belastbaren Composit gleichen,
4. die Barcolhärte den Härten der anderen Füllungsmaterialien entspricht,
5. die Wasserlöslichkeit eine Spur höher ist, dafür aber auch eine höhere Fluoridfreisetzung bei hoher physikalischer Festigkeit im Vergleich mit Compositen aufweist (siehe folgend).

Die wichtige Eigenschaft der bioaktiven Freisetzung von Ionen konnte anhand der starken Fluoridfreisetzung des C MH/CaF, im Vergleich zu dem CaF₂-freien Vergleichskomposit C MH und dem Compomer und dem bekanntermaßen deutlich fluoridfreisetzenden "resinreinforced cement" dargelegt werden.

Dazu wurden 4 Probekörper eines Durchmessers von 2 cm und einer Dicke von 2 mm mit einer Gesamtoberfläche von 45 cm² in 100 mL Wasser plaziert und die Fluoridfreisetzung gemessen.

Es wurden Messungen im Bereich von 0 - 90 Tagen durchgeführt.

Die Ergebnisse sind im Diagramm 1 wiedergegeben.

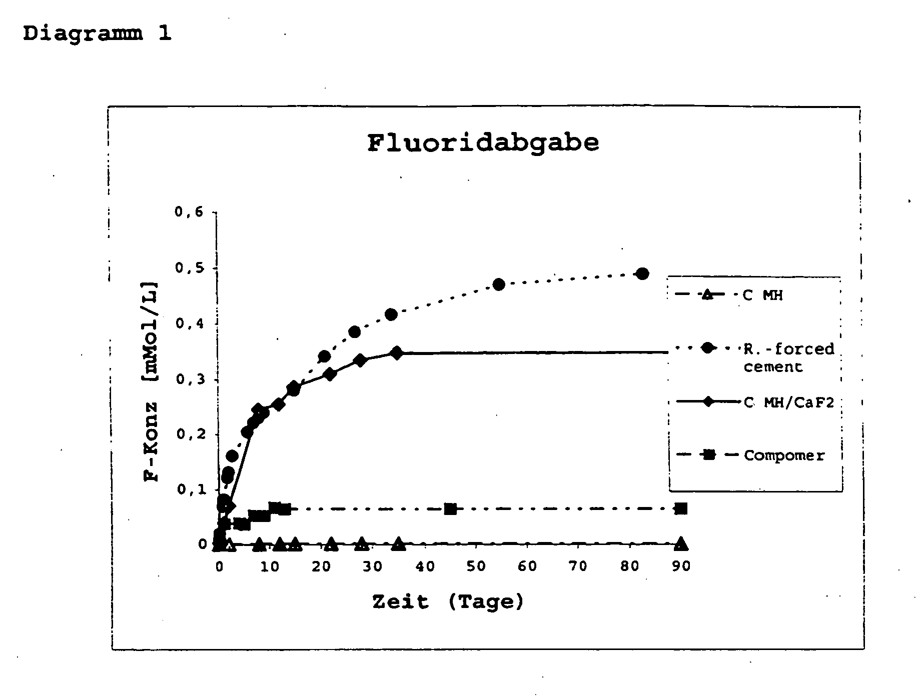

Aus dem dargestellten Diagramm der Fluoridfreisetzung, läßt sich folgern, dass
1. die Größenordnung der Fluoridfreisetzungsrate in ähnlicher Größenordnung wie die Fluoridfreisetzung der "re-inforced cements" liegt oder liegen_kann,
2. die Größenordnung der Fluoridfreisetzungsrate höher liegt als bei dem geprüften Compomeren, die einen lichthärtbaren Einkomponenten-Glasionomerzement in Pastenform darstellen,
3. die Compositformulierung ohne Nano-Calciumfluorid (C MH), die den Stand der Technik von Compositen darstellt, nahezu keine oder extrem geringe Fluorid-Freisetzung bzw. -Freisetzungsrate zeigt.

### Beispiel 3:

Als Beispiel einer Verwendung von nanokristallinen Calciumfluorid in einem lichthärtbaren selbst-ätzenden Veneers Zement wurde folgende Paste hergestellt:

| | |
|---|---|
| 47,5 | Teile Artegral One (Fa. Merz Dental, Deutschland) (selbst-ätzendes Einkomponenten-Adhäsiv) |
| 40,7 | Teile Bariumaluminiumborsilikatglas 0,6 µ, methacrylsilanisiert |
| 11,8 | Teile nanokristallienes Calciumfluorid aus Beispiel 1. |

Aus obigen Komponenten wurde eine Paste hergestellt und mit Hilfe einer Halogenlampe (Translux EC von Kulzer Deutschland) für die Untersuchung von verschiedenen Eigenschaften und Hafttests entsprechende Probekörper aus der erhaltenden Paste gefertigt.

Die Ergebnisse der Messungen der physikalischen Eigenschaften an lichtpolymerisierten Prüfkörpern gibt die Tabelle 2 wieder:

**Tabelle 2**

| | **Lichthärtbarer selbst-ätzender Veneers Zement** |
|---|---|
| Durchhärtetiefe 40sec Bestrahlung [mm] | 6,60 |
| Opazität [%] | 75,9 |
| Biegebruchfestigkeit [MPa] | 147,5 |
| Elastizitätsmodul [GPa] | 5,568 |
| Barcolhärte nach 10 min | 70/70/71 |
| Selbst-Haftkraft vom Composite-Veneer auf Schmelz N/mm² | 18,0 |

Die oben angeführte Formulierung eignet sich hervorragend als lichthärtender selbstätzender Kleber für Composite-oder methacrylsilanisierten Keramik-Facetten auf Zahnschmelz.

Auch für ein schonendes Befestigen von Modeschmuck auf Zähnen (Twinkles) oder orthodontischen Brackets ist der Klebezement hervorragend geeignet, da seine Fluoridfreisetzungskapazität die pflegegestörte Zone um diese Objekte besonders Fluorid-härten kann.

### Beispiel 4.

Als Beispiel einer Verwendung von nanokristallinen Calciumfluorid in ringöffnendenden Monomer-Mischungen wurde folgender dünnfließender trocken-härtende Zahnlack hergestellt:

| | |
|---|---|
| 75,6 Teile | Silbione UV Polymer 30 (Rhodia, Frankreich) |
| 4,1 Teile | Rhodosil Photosensitizer (Rhodia) |
| 11,2 Teile | Silbione UV PI + (Rhodia) |
| 9,1 Teile | Calciumfluorid aus Beispiel 1 |

Der erhältene mit Calciumfluorid gefüllte Lack kann sehr dünn und nahezu unsichtbar-transparent auf den Zahn aufgetragen und mit Hilfe einer Halogenlampe (Translux EC von Kulzer Deutschland) gehärtet werden. Der gehärtete Lack ohne Schmierschicht schützt den Zahn mechanisch und durch Fluoridhärtung.

Für die Untersuchung der Opazität wurden 2 mm dicke Probekörper aus der erhaltenden dünnfließenden Lack-Paste hergestellt.

Gegenüber dem ungefüllten Harz-System zeigt die mit nanokristallinen Calciumfluorid gefüllte Harzmatrix eine erstaunlich geringe Opazitätsveränderung (die Opazität von Füllungsmaterialien liegt bei 60 - 90%).

Opazität des ungefüllten Harzes: 19,6%.

Opazität des mit 9,1% Calciumfluorid gefüllten Harzes: 43,8%.

### Beispiel 5.

Ein weiteres Beispiel einer Verwendung von nanokristallinen Calciumfluorid ist die Herstellung eines gefüllten selbst-ätzenden Adhäsives für Restaurationen mit lichthärtbaren und selbsthärtenden Komposit- und Kompomer-Füllungsmaterialien. Dazu wurde folgendes modifiziertes Adhesiv hergestellt:

| | |
|---|---|
| 95 | Teile "Autobond" Teil A (Fa. Apol,France) |
| 5 | Teile nanokristallines Calciumfluorid aus Beispiel 1 |

Das erhaltene modifizierte, leicht mit Nano-Calciumfluorid gefüllte selbstätzende Adhäsiv wurde mit dem unmodifizierten Teil B des ",Autobond"s gemischt und nach der Gebrauchsinformation des Herstellers auf den Zahn mit lichthärtbarem Composit "Apol" appliziert.

Die Haftwerte von 16,29 MPa auf Schmelz und 12,57 MPa auf Dentin liegen in der Größenordnung des unmodifizierten Autobond-Systems. Im Gegensatz zum Autobond-System besitzt das neue mit Calciumfluorid gefüllte Adhäsiv aber die Fähigkeit, FluoridIonen abzugeben und damit die behandelte Zahnhartsubstanz zu festigen.

## Patentansprüche

1. Härtbare Restaurationsmaterialien für Zähne basierend auf mindestens einem vernetzbaren Harz-System und / oder mindestens einem härtbaren Säure-/Base-Zement-System, **dadurch gekennzeichnet, daß** sie mindestens ein nanostrukturiertes Erdalkalifluorid enthalten, wobei die Partikelgröße des Erdalkalifluorids mehrheitlich im Bereich von 2 bis 200 nm liegt; und
daß das vernetzbare Harz-System Monomere umfasst, die radikalisch polymerisierbar sind, oder Monomere, die unter Ringöffnung polymerisierbar sind, oder Monomere, die vernetzbare Silikone sind; und daß das härtbare Säure-Base-Zement-System ein Polyalkenoat-Zement ist, oder
daß das härtbare Säure-Base-Zement-System ein Silikat-Zement ist, oder
daß das härtbare Säure-Base-Zement-System ein Phosphat-Zement ist, oder
daß das härtbare Säure-Base-Zement-System auf Zinkoxid und/oder anderen Metalloxiden und/oder schwachen organischen Säuren und/oder Phenol-Derivaten basiert. .

2. Härtbare Restaurationsmaterialien nach Anspruch 1, **dadurch gekennzeichnet, daß** die Monomeren (Meth)-Acrylate sind.

3. Härtbare Restaurationsmaterialien nach Anspruch 1, **dadurch gekennzeichnet, daß** die Monomeren Epoxide sind.

4. Härtbare Restaurationsmaterialien nach Anspruch 1 **dadurch gekennzeichnet, daß** die Monomeren additionsvernetzende Vinylsilikone sind.

5. Härtbare Restaurationsmaterialien nach Anspruch 1, **dadurch gekennzeichnet, daß** das Erdalkalifluorid Calciumfluorid ist, oder
daß das Erdalkalifluorid Strontiumfluorid ist.

6. Härtbare Restaurationsmaterialien nach einem der vorstehenden Ansprüche, **dadurch gekennzeichnet, daß** das Erdalkalifluorid oberflächenbehandelt ist.

7. Härtbare Restaurationsmaterialien nach Anspruch 6, **dadurch gekennzeichnet, daß** das Erdalkalifluorid mit Estern der Phosphor-, Phosphon- oder mit Estern von Carbonsäuren oberflächenbehandelt ist.

8. Härtbare Restaurationsmateralien nach einem der Ansprüche 1 - 7, **dadurch gekennzeichnet, daß** sie zusätzliche Füllstoffe aus SiO₂ und/oder Glaskeramik, und/oder Gläsern und/oder Mikrokugeln und/oder anorganischen Fasern und/oder organischen Fasern enthalten.

9. Härtbare Restaurationsmaterialien nach einem der Ansprüche 1 - 8, **dadurch gekennzeichnet, daß** der Gesamtfüll-stoffgehalt aus Erdalkalifluörid und zusätzlichen Füllern 1 - 95 Gew.-% beträgt.

10. Verfahren zur Herstellung von härtbaren Restaurationsmaterialien nach einem der Ansprüche 1 - 9, **dadurch gekennzeichnet, daß** mindestens ein vernetzbares Harz-System und/oder mindestens ein härtbares Säure-Base-Zement-System, welches Erdalkalifluoride und optional zusätzliche Füllstoffe enthält, ausgehärtet, zu einer Partikelgröße zwischen 0,2 µm und 100 µm feingemahlen und anschließend als Füllstoff wieder eingesetzt wird.

## Claims

1. Curable restoration materials for teeth based on at least one crosslinkable resin system and/or at least one curable acid/base cement system, **characterised in that** they contain at least one nanostructured alkaline earth fluoride, the particle size of the alkaline earth fluoride being predominantly in the range of from 2 to 200 nm; and **in that** the crosslinkable resin system comprises monomers that are free radically polymerisable, or monomers that are polymerisable with ring-opening, or monomers that are crosslinkable silicones; and **in that** the curable acid/base cement system is a polyalkenoate cement, or **in that** the curable acid/base cement system is a silicate cement, or **in that** the curable acid/base cement system is a phosphate cement, or **in that** the curable acid/base cement system is based on zinc oxide and/or other metal oxides and/or weak organic acids and/or phenol derivatives.

2. Curable restoration materials according to claim 1, **characterised in that** the monomers are (meth)acrylates.

3. Curable restoration materials according to claim 1, **characterised in that** the monomers are epoxides.

4. Curable restoration materials according to claim 1, **characterised in that** the monomers are addition-crosslinking vinyl silicones.

5. Curable restoration materials according to claim 1, **characterised in that** the alkaline earth fluoride is calcium fluoride, or **in that** the alkaline earth fluoride is strontium fluoride.

6. Curable restoration materials according to any one of the preceding claims, **characterised in that** the alkaline earth fluoride is surface-treated.

7. Curable restoration materials according to claim 6, **characterised in that** the alkaline earth fluoride is surface-treated with esters of phosphoric acids, phosphonic acids or with esters of carboxylic acids.

8. Curable restoration materials according to any one of claims 1 to 7, **characterised in that** they contain additional fillers of SiO₂ and/or glass ceramics, and/or glasses and/or microbeads and/or inorganic fibres and/or organic fibres.

9. Curable restoration materials according to any one of claims 1 to 8, **characterised in that** the total filler content of alkaline earth fluoride and additional fillers is from 1 to 95 % by weight.

10. Process for the preparation of curable restoration materials according to any one of claims 1 to 9, **characterised in that** at least one crosslinkable resin system and/or at least one curable acid/base cement system which contains alkaline earth fluorides and optionally additional fillers is cured, finely ground to a particle size of from 0.2 µm to 100 µm and then used again as filler.

## Revendications

1. Matériaux de restauration durcissables pour les dents basés sur au moins un système de résine réticulable et/ou au moins un système de ciment acide/base durcissable, **caractérisés en ce qu'**ils contiennent au moins un fluorure alcalino-terreux nanostructuré, la taille particulaire du fluorure alcalino-terreux se situant dans le domaine de 2 à 200 nm ; et
que le système de résine réticulable comporte des monomères qui peuvent être polymérisés par voie radicalaire, ou des monomères qui peuvent être polymérisés par ouverture de cycles, ou des monomères qui sont des silicones réticulables ; et
que le système de ciment acide/base durcissable est un ciment polyalcènoate, ou
que le système de ciment acide-base durcissable est un système silicate, ou
que le système de ciment acide-base durcissable est un ciment de phosphate, ou
que le système de ciment acide-base durcissable est basé sur de l'oxyde d'étain et/ou d'autres oxydes métalliques et/ou des acides organiques faibles et/ou des dérivés de phénol.

2. Matériaux de restauration durcissables selon la revendication 1 **caractérisés en ce que** les monomères sont des (méth)-acrylates.

3. Matériaux de restauration durcissables selon la revendication 1 **caractérisés en ce que** les monomères sont des époxydes.

4. Matériaux de restauration durcissables selon la revendication 1 **caractérisés en ce que** les monomères sont des vinyl-silicones réticulables par addition.

5. Matériaux de restauration durcissables selon la revendication 1 **caractérisés en ce que** le fluorure alcalino-terreux est du fluorure de calcium, ou
que le fluorure alcalino-terreux est du fluorure de strontium.

6. Matériaux de restauration durcissables selon l'une des revendications précédentes **caractérisés en ce que** le fluorure alcalino-terreux est traité en surface.

7. Matériaux de restauration durcissables selon la revendication 6 **caractérisés en ce que** le fluorure alcalino-terreux est traité en surface avec des esters des acides phosphorique, phosphonique ou avec des esters d'acides carboxyliques.

8. Matériaux de restauration durcissables selon l'une des revendications de 1 à 7 **caractérisés en ce qu'**ils contiennent des charges complémentaires à base de SiO₂ et/ou de céramique de verre, et/ou de verres et/ou de microbilles et/ou de fibres inorganiques et/ou de fibres organiques.

9. Matériaux de restauration durcissables selon l'une des revendications de 1 à 8 **caractérisés en ce que** la teneur totale en charges est à base de fluorure alcalino-terreux de 1-95 % et d'autres charges.

10. Procédé de fabrication de matériaux de restauration durcissables selon l'une des revendications de 1 à 9 **caractérisé en ce qu'**au moins un système de résine réticulable et/ou au moins un système de ciment acide-base durcissable, qui contient un fluorure alcalino-terreux et éventuellement des charges supplémentaires, est durci, réduit en poudre jusqu'à une taille particulaire entre 0,2 µm et 100 µm et ensuite est réemployé en tant que charge.
